# FASCICULE DE BREVET EUROPEEN

(11) **EP 2 831 778 B1**
(45) Date de publication et mention de la délivrance du brevet: **04.09.2019**
(21) Numéro de dépôt: 13713423.5
(22) Date de dépôt: 27.03.2013
(51) Int. Cl.: G16H 20/13

(54) **PROCÉDÉ DE QUALIFICATION DE CONTENEUR, ENCEINTE DE STOCKAGE ASSOCIÉE**
CONTAINERQUALIFIKATIONSVERFAHREN UND ZUGEHÖRIGER LAGERRAUM
CONTAINER QUALIFICATION METHOD, ASSOCIATED STORAGE ENCLOSURE

(30) Priorité: 30.03.2012 FR 1252942
(43) Date de publication de la demande: 04.02.2015
(73) Titulaire: BIOLOG-ID, 75008 Paris (FR)
(72) Inventeur: MONGRENIER, Jean-Claude, F-78100 Saint-Germain-en-Laye (FR); DUSSAUX, François, F-77220 Gretz Armainvilliers (FR)
(74) Mandataire: Lavoix
(86) Numéro de dépôt international: PCT/EP2013/056630
(87) Numéro de publication internationale: WO 2013/144262

(56) Documents cités:
- WO-A1-00/45331
- WO-A1-2006/035465
- FR-A1- 2 825 637
- US-A1- 2003 072 676

## Description

La présente invention concerne un procédé de qualification d'un conteneur doté de premiers moyens de télécommunication sans fil comprenant une mémoire.

De tels conteneurs sont par exemple des poches contenant des produits biologiques tels que des produits sanguins (poches de sang primaire, de plasma, de plaquette, de globules rouges...) ou des produits d'ingénierie cellulaire (cellules souches...), ou encore des poches de médicaments telles que des poches de chimiothérapie.

Il est capital de pouvoir tracer les poches, et d'y associer un certain nombre d'informations (identification et dates clés relatives au contenu ...).

Il est notamment important de garder trace des informations permettant de qualifier ou déqualifier les poches en fonction de l'âge de la poche et des conditions atmosphériques de stockage, parmi lesquelles température, pression, humidité. En effet, le vieillissement des poches varie selon ces conditions de stockage.

Le document EP 1 397 104 décrit une technique selon laquelle lorsqu'une poche de sang est stockée dans une enceinte à atmosphère contrôlée, il est procédé régulièrement à un calcul d'un indice de vieillissement actualisé en fonction d'une température moyenne dans l'enceinte et du temps écoulé depuis la dernière actualisation, L'indice de vieillissement actualisé est ensuite inscrit dans l'étiquette RFID de la poche de sang, avec la date et l'heure de l'actualisation. Il y est en outre imposé qu'un indice de vieillissement actualisé soit inscrit dans l'étiquette RFID de cette poche, à chaque entrée/sortie d'une poche enceinte à atmosphère contrôlée.

Il est toutefois besoin d'une technique permettant de détecter des ruptures dans le processus de stockage des poches afin le cas échéant de déqualifier des poches dont la continuité des conditions atmosphériques de stockage n'est pas garantie.

A cet effet, selon l'invention, un procédé du type précité est caractérisé en ce qu'il comprend les étapes suivantes lorsque le conteneur est stocké dans une enceinte de stockage de conteneurs, ladite enceinte comprenant au moins un capteur de conditions atmosphériques adapté pour mesurer au sein de l'enceinte au moins un paramètre atmosphérique parmi la température, la pression et l'humidité, et au moins des seconds moyens de télécommunications sans fil :
a/ émission radiofréquence par les seconds moyens de télécommunications sans fil de l'enceinte, à destination des premiers moyens de télécommunication sans fil du conteneur, d'un message comprenant une valeur représentative d'un résultat d'une mesure dudit paramètre dans l'enceinte; l'étape a/ étant réitérée après chaque instant de mesure d'une pluralité d'instants de mesure successifs déterminés ;
b/ pour chacun desdits messages reçus par les premiers moyens de télécommunication sans fil du conteneur, mémorisation, par lesdits premiers moyens de télécommunication sans fil du conteneur, de la valeur représentative comprise dans ledit message reçu, la mémoire des premiers moyens de télécommunication sans fil du conteneur comportant des valeurs représentatives de résultat de mesure dudit paramètre successivement mémorisées associées chacune à un instant de mesure déterminé distinct ;
selon lequel le procédé comprend en outre une étape de qualification ou déqualification du conteneur en fonction d'au moins une comparaison entre le nombre de valeurs représentatives d'un résultat d'une mesure dudit paramètre mémorisées dans la mémoire des premiers moyens de télécommunication sans fil du conteneur et le nombre desdits instants de mesure déterminés.

Un tel procédé permet d'accroître la garantie de la présence continue d'un conteneur dans l'enceinte du conteneur et de détecter les discontinuités tant dans la présence du conteneur dans l'enceinte que dans les mesures de températures.
Dans des modes de réalisation, le procédé suivant l'invention comporte en outre une ou plusieurs des caractéristiques suivantes :
- les premiers moyens de télécommunication sans fil du conteneur comportent une étiquette RFID et les seconds moyens de télécommunications sans fil de l'enceinte comportent un lecteur RFID ;
- à l'étape a/, le message comprend en outre une indication de l'instant de ladite mesure déterminé et à l'étape b/, ladite indication est mémorisée en correspondance avec ladite valeur représentative ;
- les étapes a/ et b/ sont réitérées chaque période de longueur T ;
- T est compris entre 10 secondes et 6 heures ;
- le conteneur est qualifié ou déqualifié en fonction d'un nombre d'instants de mesure déterminés pour lesquelles la mémoire des premiers moyens de télécommunication sans fil du conteneur ne comporte pas de valeur représentative d'un résultat de mesure, lesdits instants de mesure étant situés entre deux instants de mesure pour lesquels des valeurs représentatives d'un résultat ont été mémorisés dans la mémoire des premiers moyens de télécommunication ;
- un conteneur est qualifié ou déqualifié en outre en fonction des valeurs représentatives mémorisées dans la mémoire des premiers moyens de télécommunication sans fil du conteneur ;
- le procédé comprend les étapes suivantes lorsque le conteneur est stocké dans une enceinte de transport de conteneurs, puis transporté entre un point de départ et un point de destination dans ladite enceinte de transport, ladite enceinte de transport comprenant un capteur adapté pour mesurer la valeur d'au moins un paramètre atmosphérique parmi la température, la pression, l'humidité au sein de l'enceinte et des troisièmes moyens de télécommunications sans fil comprenant une mémoire :
   - enregistrement, pendant le transport, pour chacun de plusieurs instants de mesure successifs déterminés, dans la mémoire des troisièmes moyens de télécommunication sans fil de l'enceinte de transport, d'une valeur représentative d'un résultat d'une mesure dudit paramètre ;
   - émission, par les troisièmes moyens de télécommunication sans fil de l'enceinte de transport, desdites valeurs représentatives vers un dispositif de traitement comprenant des quatrièmes moyens de télécommunication sans fil et situé au point de destination ;
   - qualification ou déqualification du conteneur en fonction desdites valeurs représentatives reçues par le dispositif de traitement ;
- des troisièmes moyens de télécommunications sans fil comprennent une étiquette RFID et les quatrièmes moyens de télécommunication sans fil comprennent un lecteur RFID ; et
- le procédé comprend en outre les étapes suivantes :
   - émission, depuis les quatrièmes moyens de télécommunication sans fil du dispositif de traitement à destination des premiers moyens de communication sans fil du conteneur, d'au moins lesdites valeurs représentatives reçues par le dispositif de traitement ;
   - réception desdites valeurs représentatives par les premiers moyens de communication sans fil du conteneur et mémorisation par lesdits premiers moyens de communication desdites valeurs de paramètre reçues dans la mémoire.

Suivant un deuxième aspect, la présente invention propose une enceinte de stockage de conteneurs comprenant au moins un capteur de conditions atmosphériques adapté pour mesurer au sein de l'enceinte au moins un paramètre atmosphérique parmi la température, la pression et l'humidité, et ladite enceinte comprenant des seconds moyens de télécommunication sans fil adaptés pour communiquer avec des premiers moyens de télécommunication sans fil d'au moins un conteneur stocké dans l'enceinte, ladite enceinte de stockage étant caractérisée en ce qu'elle est adaptée pour émettre, après chaque instant de mesure d'une pluralité d'instants de mesures successifs déterminés, à destination des premiers moyens de télécommunication sans fil d'au moins ledit conteneur, un message comprenant une valeur représentative d'un résultat d'une mesure dudit paramètre dans l'enceinte en vue de la mémorisation par les premiers moyens de télécommunication sans fil, de ladite valeur représentative et en ce qu'elle est adaptée pour qualifier ou déqualifier un conteneur en fonction d'au moins une comparaison entre le nombre de valeurs représentatives d'un résultat d'une mesure dudit paramètre mémorisées dans la mémoire des premiers moyens de télécommunication sans fil du conteneur et le nombre desdits instants de mesure déterminés.

Ces caractéristiques et avantages de l'invention apparaîtront à la lecture de la description qui va suivre, donnée uniquement à titre d'exemple, et faite en référence aux dessins annexés, sur lesquels :
- la figure 1 représente une enceinte de stockage de conteneurs dans un mode de réalisation de l'invention ;
- la figure 2 représente un emplacement de stockage de l'enceinte de la figure 1 ;
- la figure 3 représente une poche de sang considéré dans un mode de mise en oeuvre de l'invention ;
- la figure 4 est un organigramme d'un procédé dans un mode de réalisation de l'invention ;
- la figure 5 représente un coffre de transport..

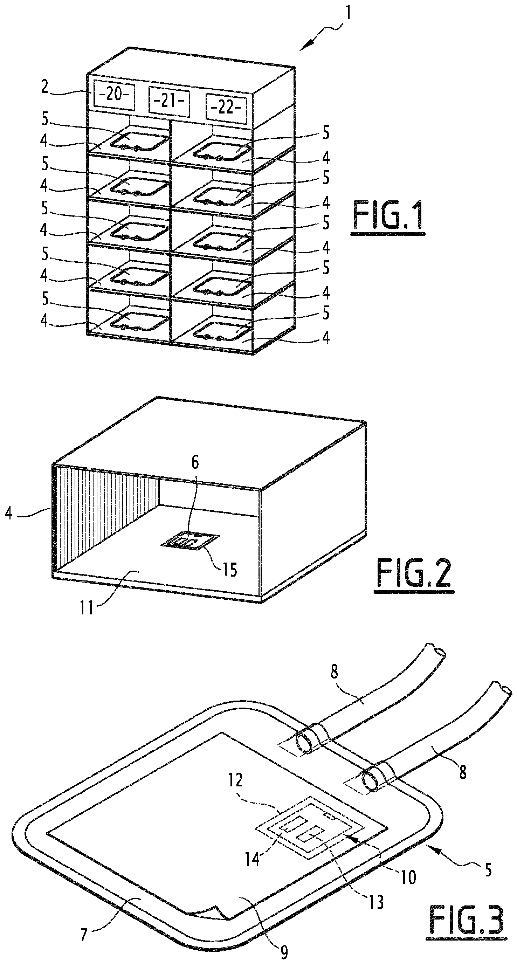

Sur la figure 3, une poche 5 est représentée, destinée, dans le cas présent, à contenir du sang.

De façon connue, cette poche 5 est un contenant étanche de sang en matière plastique respirante permettant le métabolisme, du type PVC (polychlorure de vinyle), polycarbonate ou PEG (polyéthylène glycol).

La poche 5 comporte des tubulures 8 obturées, par exemple par soudure. Ces tubulures 8 ont été utilisées, avant leur obturation, pour introduire le sang dans la poche 5.

Une étiquette adhésive 9 est collée sur une face extérieure de la poche 5. Cette étiquette adhésive 9 comporte une puce RFID 10.

La puce RFID 10 comporte une antenne radiofréquence 12, une mémoire 13 et éventuellement un microprocesseur 14.

Sur la figure 1 est représentée une enceinte de stockage 1 à atmosphère contrôlée destinée à stocker des poches de sang similaires à la poche de sang 5 représentée en figure 3.

L'enceinte 1 comporte un étage de pilotage 2, comportant un microcontrôleur 21, une mémoire 22 et un thermomètre 20.

Le thermomètre 20 est adapté pour mesurer la température courante au sein de l'enceinte 1.

L'enceinte 1 comporte aussi des moyens, non représentés, destinés à la ventilation et à réguler la température en fonction de la température couramment mesurée, de manière à maintenir la température au sein de l'enceinte 1 dans une plage donnée, par exemple [2°C, 6°C], où °C représente l'unité Celsius.

L'enceinte 1 comprend en outre des emplacements 4 destinés au stockage de poches de sang 5. Dans le cas considéré, une poche de sang est stockée par emplacement. Dans d'autres modes de réalisation, plusieurs poches sont stockées dans un emplacement 4.

La figure 2 représente plus en détail un emplacement 4.

Un emplacement 4 comporte un support horizontal 11 sur lequel une poche 5 à stocker est déposée. Le support 11 comporte un lecteur RFID 6 doté d'une antenne15 et adapté pour communiquer par voie radiofréquence avec une poche de sang 5 disposée dans l'emplacement 4 (la poche 5 étant disposée de préférence de manière à ce que sa face comportant l'étiquette RFID 10 soit en contact avec le lecteur RFID 6 de l'emplacement 4).

Un programme P1 stocké dans la mémoire 22 de l'enceinte 1 comprend des instructions logicielles adaptées pour mettre en oeuvre les étapes suivantes mises en oeuvre par l'enceinte lorsque le programme P1 est exécuté par le microcontrôleur 21 de l'enceinte 1.

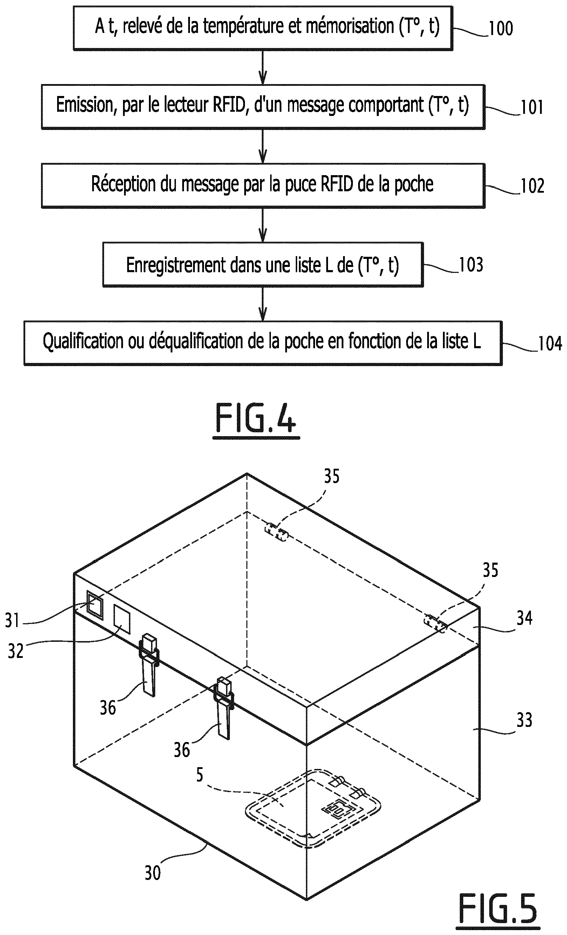

Les étapes suivantes sont mises en oeuvre toutes les périodes de longueur T1 par l'étage de pilotage 2 de l'enceinte 1 (cf. figure 4) :
- relevé de la température indiquée par le thermomètre 20 et stockage de la température relevée avec la date et l'heure du relevé dans la mémoire 22 de l'enceinte 1 (étape 100) ;
- commande au lecteur RFID de chaque emplacement 4 (éventuellement seulement au lecteur RFID de chaque emplacement occupé lorsque l'étage de pilotage stocke une liste des emplacements occupés) de mettre en oeuvre l'étape 101 suivante ;
- émission par le lecteur RFID 6 de chacun de ces emplacements 4, via son antenne 15 à destination de la poche 5, d'un message comportant la température relevée, ainsi que la date et l'heure du relevé (étape 101) et commandant l'enregistrement dans la mémoire 13 de la puce RFID 10 de la poche de ces valeurs associées de température, date et heure.

(On rappelle que de façon connue, le lecteur RFID émet une énergie à travers son antenne destinée à activer et alimenter une puce RFID ou « étiquette » RFID à proximité, et permettre l'écriture de données dans la mémoire de la puce RFID, ou l'envoi par la puce RFID d'informations.)

Les étapes suivantes sont ensuite mises en oeuvre par une poche 5 située dans un de ces emplacements, comme représenté en figure 4 :
- réception, par la puce RFID 10 de la poche 5 via son antenne 12, du message émis par le lecteur RFID 6 de l'emplacement 4 dans lequel la poche 5 est localisée (étape 102) ;
- enregistrement, dans une liste de données L mémorisée en mémoire 13 de la poche 5, d'un ensemble comportant la température relevée, la date et l'heure du relevé (étape 103), à la suite d'ensembles précédemment mémorisés la cas échéant, comportant une température relevée avec les date et l'heure du relevé.

Dans un mode de réalisation, T1 est compris dans la plage de valeurs suivante [30 s, 60 s], « s » indiquant l'unité « seconde ».

Dans un autre mode de réalisation, T1 est compris dans la plage de valeurs suivante [30 s, 10 min], « min » indiquant l'unité « minute ».

Dans un autre mode de réalisation, T1 est compris dans la plage de valeurs suivante [30 s, 6 h], « h » indiquant l'unité « heure ».

La valeur de T1 est choisie en fonction de l'effet des variations de température sur le produit contenu dans la poche et des contraintes atmosphériques de stockage.

Par exemple dans le cas présent où les poches contiennent du sang, T1 est choisie égale à 10 min.

Ainsi une poche de sang 5 stockée entre un instant Td et un instant Tf comportera nominalement, dans la liste de données L stockée dans sa puce RFID 10, les n+1 ensembles suivants (Temp_{Td+dt+iT1} étant la température relevée à l'instant Td + dt+iT1) :
(Td + dt + iT1, Temp_{Td+dt+iT1}) avec i entier égal variant de 0 à n, avec dt ≤T1 et 0≤ Tf-(Td + dt + nT1) ≤ T1.

Ainsi par analyse de la liste L de données stockée dans la puce RFID 10 d'une poche 5 ayant séjourné dans l'enceinte 1 entre les instants connus Td et Tf, une discontinuité concernant ce stockage de la poche 5 dans l'enceinte 1 est détectable lorsque la liste ne comporte pas les ensembles nominalement présents tels qu'indiqués ci-dessus.

Ainsi l'absence d'un ou de plusieurs ensemble(s) de données (Td + dt + iT1 , Temp_{Td+dt+iT1}) peut révéler une discontinuité dans le stockage : par exemple la poche 5 était hors de l'enceinte 1 au moment où elle aurait dû recevoir cet ou ces ensemble(s) de données. Ou alors, une panne dans la fourniture de l'énergie électrique à l'enceinte au moment où l'enceinte aurait dû transmettre via ses lecteurs RFID cet ou ces ensemble(s) de données a pu occasionner.

Il est donc possible, en cas d'absence d'un ensemble de données ou de k ensembles consécutifs (Td + dt + iT1 , Temp_{Td + dt+iT1}),..., (Td + dt + (i+k-1)T1 , Temp_{Td+dt+(i+k-1)T1}), notamment avec 0<i<n et 0≤k<n-i+1, de déterminer la durée maximale correspondant pendant laquelle on ne peut garantir la présence de la poche dans l'enceinte, qui est de (k -i +2)T1.

Puis en fonction, de cette durée maximale pendant laquelle la présence de la poche 5 dans l'enceinte 1 dans des contraintes de stockage requises n'est pas garantie, et en fonction en outre optionnellement de la température Temp_{Td+dt+(i-1)T1} relevée à l'instant précédant l'absence de données et/ou de la température Temp_{Td + dt+(i+k)T1} relevée à l'instant suivant l'absence de données, la poche 5 est qualifiée ou déqualifiée quant à son utilisation pour une future transfusion par exemple, dans une étape 104.

Par ailleurs, la qualification/déqualification de la poche 5 est effectuée en fonction en outre des valeurs de températures enregistrées dans la puce RFID 10 de la poche 5, éventuellement moyennées sur le temps de présence dans l'enceinte 1.

Cette opération visant à la qualification ou déqualification peut être mise en oeuvre par un dispositif informatique couplé à un lecteur RFID extérieur à l'enceinte 1, interrogeant la puce RFID 10 de la poche 5 une fois extraite de l'enceinte 1.

Dans un mode de réalisation, cette opération de qualification ou déqualification est effectuée par l'étage de pilotage 2 de l'enceinte 1 par l'intermédiaire du lecteur RFID 6 de l'emplacement 4 de stockage de la poche 5, par exemple à des instants réguliers et/ou à l'occasion de la sortie hors de l'enceinte 1 de la poche 5, détectée par l'enceinte 1 elle-même ou signalée à l'enceinte 1 par un opérateur interagissant avec l'étage de pilotage 2 de l'enceinte 1.

Dans un autre mode de réalisation, le résultat de l'opération de qualification/déqualification est mémorisé dans la puce RFID 10 de la poche 5 à l'aide du lecteur RFID utilisé pour effectuer cette opération.

Dans un mode de réalisation de l'invention, le message envoyé par le lecteur RFID 6 à l'étape 101 comporte à la place de la date et heure du relevé, un identifiant de l'heure et date du relevé, par exemple une position dans la mémoire 22 de l'enceinte 1 correspondant à la date et heure du relevé.

Dans un mode de réalisation de l'invention, le message envoyé par le lecteur RFID 6 à l'étape 101 comporte à la place de la température relevée, un indicateur. La valeur de cet indicateur est déterminée par l'étage de pilotage 2 en fonction de la température couramment mesurée, et peut prendre par exemple 2 valeurs distinctes (les valeurs 0 et 1, ou OK et KO). La valeur courante est par exemple 0, ou KO, quand la température couramment mesurée n'est pas dans la plage donnée, cette plage étant par exemple [+2°C, +6°C]. La valeur courante est par exemple 1 ou OK quand la température est dans la plage donnée.

Dans un mode de réalisation, le message ne comporte pas de date/heure de relevé, ni d'identifiant de date/heure. Dans un tel cas, la qualification/déqualification est réalisée en fonction de la vérification du nombre de valeurs effectivement mémorisées dans la puce RFID 10 de la poche 5, de température ou d'indicateurs relatifs aux valeurs de température comparées au nombre de valeurs qui aurait dû théoriquement être mémorisé d'après la fréquence des commandes d'inscription, régulière ou non, en étape 101 pendant la période de stockage de la poche dans l'enceinte.

Dans un autre mode de réalisation de l'invention qui peut être mise en oeuvre indépendamment ou non des modes de réalisation décrits ci-dessus, on considère une enceinte de transport comprenant un capteur adapté pour mesurer la valeur d'au moins un paramètre atmosphérique au sein de l'enceinte, par exemple la température, et une étiquette RFID comprenant une mémoire.

Cette enceinte de transport est adaptée pour enregistrer dans la mémoire de l'étiquette RFID de l'enceinte de transport, des valeurs de paramètre successivement mesurées pendant le transport, associées à des indications temporelles représentant les instants de mesure successifs.

Dans un mode de réalisation, une poche 5 ayant séjournée ou non dans l'enceinte 1, est transportée depuis un endroit de départ jusqu'à un endroit de destination au sein d'une telle enceinte de transport, par exemple un coffre de transport 30, à atmosphère régulée ou non.

Le coffre de transport est adapté pour contenir une ou plusieurs poches 5.

Le coffre de transport 30, comme représenté en figure 5, comporte un caisson 33 recouvert d'un couvercle 34. Le caisson 33 et le couvercle 34 sont par exemple reliés par une charnière 35 permettant l'ouverture et la fermeture du couvercle par rotation du couvercle. Par ailleurs, des éléments de fixation 36 permettent de bloquer le couvercle une fois fermé sur le caisson, notamment pendant les opérations de transport, de manière à limiter les variations de température des poches contenues dans le coffre.

Le coffre de transport 30 comporte en outre un enregistreur RFID 31 comprenant un microprocesseur, une mémoire, et une antenne radiofréquence.

Le coffre de transport 30 comporte en outre un thermomètre 32 adapté pour mesurer la température dans la partie du coffre contenant la poche 5 et éventuellement d'autres poches.

Le coffre de transport 30 est adapté pour effectuer chaque période de longueur T2 (par exemple T2=T1), un relevé de la température indiquée par le thermomètre 32 du coffre et stocker successivement chaque température relevée avec la date et l'heure du relevé correspondant dans la mémoire de l'enregistreur RFID 31 le long du cheminement entre l'endroit de départ et l'endroit d'arrivée.

Quand le coffre de transport 30 est présenté au point de destination à un opérateur, ce dernier lit à l'aide d'un lecteur RFID couplé à un dispositif informatique de qualification, les températures respectivement associées aux dates et heures de relevé correspondantes qui ont été successivement mémorisées dans l'enregistreur RFID 31 du coffre de transport.

Le dispositif informatique de qualification est adapté pour mettre en oeuvre une opération de qualification/déqualification de la poche 5 et le cas échéant des autres poches transportées dans le coffre, l'opération de qualification étant similaire à celle présentée ci-dessus en étape 104, mais en fonction cette fois des températures, dates et heures de relevées extraites du coffre de transport 30.

Ainsi, il peut être procédé au refus ou à l'acceptation des poches ainsi délivrées sans même avoir à ouvrir le coffre de transport.

Dans un mode de réalisation, le résultat de l'opération de qualification/déqualification est inscrit dans la puce RFID 10 de la poche 5 par le lecteur RFID couplé au dispositif informatique, ainsi que l'heure et la date de l'opération, avec un identifiant du dispositif informatique.

Dans un mode de réalisation, quand la poche 5 est qualifiée, les températures ou indicateurs associés aux heures de relevé lues par le dispositif informatique sont également inscrites dans la puce RFID 10 de la poche 5 par le lecteur RFID couplé au dispositif informatique.

Comme dans le cas de l'enceinte 1 évoquée plus haut, de telles dispositions permettent de tester la présence quasi-continue (selon la valeur de T1 ou T2) des poches au sein de l'enceinte, en associant ces tests de présence à la température régnant alors dans l'enceinte ou le coffre.

Dans un mode de réalisation, les communications RFID mises en oeuvre entre un lecteur RFID et une puce RFID indiquées dans les modes de réalisation évoquées ci-dessus sont chiffrées.

Des modes de réalisation de l'invention ont été décrits ci-dessus en considérant le paramètre de température. Tout autre paramètre illustrant les conditions atmosphérique de stockage peut être considéré pour mettre en oeuvre l'invention à la place de la température, ou en plus.

Des modes de réalisation de l'invention ont été décrits ci-dessus en considérant des poches de sang. L'invention peut bien sûr être mise en oeuvre pour tout type de poche destinée à contenir des produits dont l'utilisation est conditionnée par des contraintes de stockage strictes. Dans un mode de réalisation, l'invention s'applique à des poches contenant des produits biologiques tels que des produits sanguins (poches de sang primaire, de plasma, de plaquettes, de globules rouges...) ou des produits d'ingénierie cellulaire (cellules humaines ou animales, notamment cellules souches humaines ou animales, produits issus de cellules humaines ou animales). Dans un autre mode de réalisation, l'invention s'applique à des poches de médicaments ou préparations thérapeutiques contenant un ou plusieurs principes actifs ou médicaments, telles que des poches de chimiothérapie (contenant généralement un soluté et un ou plusieurs principes actifs de chimiothérapie). De manière plus générale, l'invention s'applique à tout produit destiné à être perfusé à un patient (humain ou animal).

L'enceinte 1 de stockage des poches considérée en référence aux figures 1 et 2 comporte des emplacements de stockage sur lesquels les poches sont disposées horizontalement. L'invention peut bien sûr être mise en oeuvre avec toutes autres configurations de stockage.

En outre, dans l'exemple présenté, un lecteur RFID était propre à chaque emplacement : l'invention peut être mise en oeuvre dans les cas où un même lecteur RFID est utilisé pour plusieurs emplacements.

## Revendications

1. Procédé de qualification d'un conteneur (5) doté de premiers moyens de télécommunication sans fil (10) comprenant une mémoire (13), ledit procédé étant **caractérisé en ce qu'**il comprend les étapes suivantes lorsque le conteneur est stocké dans une enceinte (1) de stockage de conteneurs, ladite enceinte comprenant au moins un capteur (20) de conditions atmosphériques adapté pour mesurer au sein de l'enceinte au moins un paramètre atmosphérique parmi la température, la pression et l'humidité, et au moins des seconds moyens de télécommunications sans fil (6) :
a/ émission radiofréquence par les seconds moyens de télécommunications sans fil de l'enceinte, à destination des premiers moyens de télécommunication sans fil (10) du conteneur, d'un message comprenant une valeur représentative d'un résultat d'une mesure dudit paramètre dans l'enceinte ; l'étape a/ étant réitérée après chaque instant de mesure d'une pluralité d'instants de mesure successifs déterminés ;
b/ pour chacun desdits messages reçus par les premiers moyens de télécommunication sans fil du conteneur, mémorisation, par lesdits premiers moyens de télécommunication sans fil du conteneur, de la valeur représentative comprise dans ledit message reçu, la mémoire des premiers moyens de télécommunication sans fil du conteneur comportant des valeurs représentatives de résultat de mesure dudit paramètre successivement mémorisées associées chacune à un instant de mesure déterminé distinct ;
selon lequel le procédé comprend en outre une étape de qualification ou déqualification du conteneur (5) en fonction d'au moins une comparaison entre le nombre de valeurs représentatives d'un résultat d'une mesure dudit paramètre mémorisées dans la mémoire des premiers moyens de télécommunication sans fil (10) du conteneur et le nombre desdits instants de mesure déterminés.

2. Procédé selon la revendication 1, selon lequel les premiers moyens de télécommunication sans fil du conteneur comportent une étiquette RFID et les seconds moyens de télécommunications sans fil de l'enceinte comportent un lecteur RFID.

3. Procédé selon la revendication 1 ou 2, selon lequel à l'étape a/, le message comprend en outre une indication de l'instant de ladite mesure déterminé et selon lequel à l'étape b/, ladite indication est mémorisée en correspondance avec ladite valeur représentative.

4. Procédé selon l'une quelconque des revendications précédentes, selon lequel les étapes a/ et b/ sont réitérées chaque période de longueur T.

5. Procédé selon la revendication 4, selon lequel T étant compris entre 10 secondes et 6 heures.

6. Procédé selon l'une quelconque des revendications précédentes, selon lequel le conteneur (5) est qualifié ou déqualifié en fonction d'un nombre d'instants de mesure déterminés pour lesquelles la mémoire (13) des premiers moyens de télécommunication sans fil (10) du conteneur ne comporte pas de valeur représentative d'un résultat de mesure, lesdits instants de mesure étant situés entre deux instants de mesure pour lesquels des valeurs représentatives d'un résultat ont été mémorisés dans la mémoire des premiers moyens de télécommunication.

7. Procédé selon l'une des revendications précédentes, selon lequel un conteneur (5) est qualifié ou déqualifié en outre en fonction des valeurs représentatives mémorisées dans la mémoire des premiers moyens de télécommunication sans fil (10) du conteneur.

8. Procédé selon l'une des revendications précédentes, comprenant les étapes suivantes lorsque le conteneur (5) est stocké dans une enceinte (30) de transport de conteneurs, puis transporté entre un point de départ et un point de destination dans ladite enceinte de transport, ladite enceinte de transport comprenant un capteur (32) adapté pour mesurer la valeur d'au moins un paramètre atmosphérique parmi la température, la pression, l'humidité au sein de l'enceinte et des troisièmes moyens de télécommunication sans fil (31) comprenant une mémoire :
- enregistrement, pendant le transport, pour chacun de plusieurs instants de mesure successifs déterminés, dans la mémoire des troisièmes moyens de télécommunications sans fil (31) de l'enceinte de transport (30), d'une valeur représentative d'un résultat d'une mesure dudit paramètre ;
- émission, par les troisièmes moyens de télécommunication sans fil de l'enceinte de transport (30), desdites valeurs représentatives vers un dispositif de traitement comprenant des quatrièmes moyens de télécommunication sans fil et situé au point de destination ;
- qualification ou déqualification du conteneur en fonction desdites valeurs représentatives reçues par le dispositif de traitement.

9. Procédé selon la revendication 8, selon lequel des troisièmes moyens de télécommunications sans fil (31) comprennent une étiquette RFID et les quatrièmes moyens de télécommunication sans fil comprennent un lecteur RFID.

10. Procédé selon la revendication 8 ou 9, comprenant en outre les étapes suivantes :
- émission, depuis les quatrièmes moyens de télécommunication sans fil du dispositif de traitement à destination des premiers moyens de communication sans fil du conteneur (5), d'au moins lesdites valeurs représentatives reçues par le dispositif de traitement ;
- réception desdites valeurs représentatives par les premiers moyens de communication sans fil du conteneur et mémorisation par lesdits premiers moyens de communication desdites valeurs de paramètre reçues dans la mémoire.

11. Enceinte (1) de stockage de conteneurs, comprenant au moins un capteur (20) de conditions atmosphériques adapté pour mesurer au sein de l'enceinte au moins un paramètre atmosphérique parmi la température, la pression et l'humidité, et ladite enceinte comprenant des seconds moyens de télécommunication sans fil (6) adaptés pour communiquer avec des premiers moyens de télécommunication sans fil d'au moins un conteneur (5) stocké dans l'enceinte, ladite enceinte de stockage étant **caractérisée en ce qu'**elle est adaptée pour émettre, après chaque instant de mesure d'une pluralité d'instants de mesures successifs déterminés, à destination des premiers moyens de télécommunication sans fil d'au moins ledit conteneur, un message comprenant une valeur représentative d'un résultat d'une mesure dudit paramètre dans l'enceinte en vue de la mémorisation par les premiers moyens de télécommunication sans fil, de ladite valeur représentative ;
et **en ce qu'**elle est adaptée pour qualifier ou déqualifier un conteneur en fonction d'au moins une comparaison entre le nombre de valeurs représentatives d'un résultat d'une mesure dudit paramètre mémorisées dans la mémoire des premiers moyens de télécommunication sans fil (10) du conteneur et le nombre desdits instants de mesure déterminés.

12. Enceinte selon la revendication 11, dans laquelle les seconds moyens de télécommunications sans fil de l'enceinte comportent un lecteur RFID, les premiers moyens de télécommunication sans fil du conteneur comportant une étiquette RFID.

13. Enceinte selon la revendication 11 à 12, adaptée pour insérer dans le message une indication de l'instant de ladite mesure déterminé.

14. Enceinte selon l'une quelconque des revendications 11 à 13, dans laquelle les instants de mesure sont distants d'une période de longueur T.

15. Enceinte selon la revendication 14, dans laquelle T étant compris entre 10 secondes et 6 heures.

## Patentansprüche

1. Verfahren zum Qualifizieren eines Containers (5), ausgestattet mit ersten drahtlosen Telekommunikationsmitteln (10), die einen Speicher (13) aufweisen, wobei das Verfahren **dadurch gekennzeichnet ist, dass** es die folgenden Schritte aufweist, wenn der Container in einem Behälter (10) zur Containerlagerung gelagert ist, wobei der Behälter mindestens einen Sensor (20) für atmosphärische Bedingungen, der eingerichtet ist, innerhalb des Behälters mindestens einen atmosphärischen Parameter von der Temperatur, dem Druck und der Feuchtigkeit zu messen und mindestens zweite drahtlose Telekommunikationsmittel (6) aufweist:
a/ Funkfrequenzübertragung, durch die zweiten drahtlosen Telekommunikationsmittel des Behälters an die ersten drahtlosen Funktelekommunikationsmittel (10) des Containers, einer Nachricht, die einen Wert aufweist, der repräsentativ für ein Ergebnis einer Messung des Parameters im Behälter ist; wobei Schritt a/ nach jedem Messzeitpunkt einer Vielzahl von vorgegebenen aufeinanderfolgenden Messzeiten wiederholt wird;
b/ für jede der Nachrichten, die von den ersten drahtlosen Telekommunikationsmitteln des Containers empfangen werden, Speichern des in der empfangenen Nachricht enthaltenen repräsentativen Werts durch die ersten drahtlosen Telekommunikationsmittel des Containers, wobei der Speicher der ersten drahtlosen Telekommunikationsmittel des Containers nacheinander gespeicherte für Messergebnisse repräsentative Werte des Parameters enthält, wobei jeder einem anderen vorgegebenen Messzeitpunkt zugeordnet ist;
gemäß dem das Verfahren ferner einen Schritt des Qualifizierens oder Dequalifizierens des Containers (5) in Abhängigkeit von mindestens einem Vergleich zwischen der Anzahl von Werten, die repräsentativ sind für ein Ergebnis einer Messung des Parameters, die im Speicher der ersten Telekommunikationsmittel (10) des Containers gespeichert sind, und der Anzahl der ermittelten Messzeitpunkte aufweist.

2. Verfahren nach Anspruch 1, wobei die ersten drahtlosen Telekommunikationsmittel des Containers ein RFID-Etikett enthalten und die zweiten drahtlosen Telekommunikationsmittel des Behälters ein RFID-Lesegerät enthalten.

3. Verfahren nach Anspruch 1 oder 2, wobei in Schritt a/ die Nachricht ferner eine Angabe des vorgegebenen Messzeitpunkts aufweist und wobei in Schritt b/ die Angabe korrespondierend mit dem repräsentativen Wert gespeichert wird.

4. Verfahren nach einem der vorhergehenden Ansprüche, wobei die Schritte a/ und b/ für jede Periode der Länge T wiederholt werden.

5. Verfahren nach Anspruch 4, wobei T zwischen 10 Sekunden und 6 Stunden liegt.

6. Verfahren nach einem der vorhergehenden Ansprüche, bei dem der Container (5) in Abhängigkeit einer Anzahl von vorgegebenen Messzeitpunkten, für die der Speicher (13) der ersten drahtlosen Telekommunikationsmittel (10) keinen repräsentativen Wert eines Messergebnisses enthält, qualifiziert oder dequalifiziert wird, wobei die Messzeitpunkte zwischen zwei Messzeitpunkten liegen, für die Werte, die ein Ergebnis repräsentieren, im Speicher der ersten Telekommunikationsmittel gespeichert wurden.

7. Verfahren nach einem der vorhergehenden Ansprüche, bei dem ein Container (5) ferner in Abhängigkeit der im Speicher des ersten drahtlosen Telekommunikationsmittel (10) des Containers gespeicherten repräsentativen Werten qualifiziert oder dequalifiziert wird.

8. Verfahren nach einem der vorhergehenden Ansprüche, aufweisend die folgenden Schritte, wenn der Container (5) in einem Containertransportbehälter (30) gelagert ist und dann zwischen einem Startpunkt und einem Zielpunkt in dem Transportbehälter transportiert wird, wobei der Transportbehälter einen Sensor (32), der dazu ausgelegt ist, den Wert von mindestens einem atmosphärischen Parameter von der Temperatur, dem Druck, der Feuchtigkeit innerhalb des Behälters zu messen, und dritte drahtlose Telekommunikationsmittel (31), die einen Speicher aufweisen, aufweist:
- Aufzeichnen, während des Transports für jeden von mehreren vorgegebenen aufeinanderfolgenden Messzeitpunkten in dem Speicher der dritten drahtlosen Telekommunikationsmittel (31) des Transportbehälters (30), eines Werts, der für ein Ergebnis einer Messung des Parameters repräsentativ ist;
- Übertragen der repräsentativen Werte durch die dritten drahtlosen Telekommunikationsmittel des Transportbehälters (30) an eine Verarbeitungsvorrichtung, die vierte drahtlose Telekommunikationsmittel aufweist und sich am Zielpunkt befindet;
- Qualifizierung oder Dequalifizierung des Containers in Abhängigkeit der von der Verarbeitungsvorrichtung erhaltenen repräsentativen Werte.

9. Verfahren nach Anspruch 8, wobei die dritten drahtlosen Telekommunikationsmittel (31) ein RFID-Etikett aufweisen und die vierten drahtlosen Telekommunikationsmittel einen RFID-Leser aufweisen.

10. Verfahren nach Anspruch 8 oder 9, das ferner die folgenden Schritte aufweist:
- Übertragen, von den vierten drahtlosen Telekommunikationsmitteln der Verarbeitungsvorrichtung zu der ersten drahtlosen Einrichtung des Containers (5), zumindest der genannten repräsentative Werte, die von der Verarbeitungsvorrichtung empfangen wurden;
- Empfang der repräsentativen Werte durch die ersten drahtlosen Kommunikationsmittel des Containers und Speichern der empfangenen Parameterwerte in dem Speicher durch die ersten Kommunikationsmittel.

11. Containerlagerungsbehälter (1), der mindestens einen Sensor (20) für atmosphärische Bedingungen aufweist, der eingerichtet ist, innerhalb des Behälters mindestens einen atmosphärischen Parameter von Temperatur, Druck und Feuchtigkeit zu messen, und wobei der Behälter zweite drahtlose Telekommunikationsmittel (6) aufweist, die zur Kommunikation mit den ersten drahtlosen Telekommunikationsmitteln mindestens eines in dem Behälter gelagerten Containers (5) eingerichtet sind, wobei der Lagerungsbehälter **dadurch gekennzeichnet ist, dass** er eingerichtet ist, nach jedem Messzeitpunkt mehrerer aufeinanderfolgender Messzeitpunkte, an die ersten drahtlosen Telekommunikationsmittel mindestens des Containers eine Nachricht, die einen Wert enthält, der für ein Ergebnis einer Messung des Parameters in dem Lagerungsbehälter repräsentativ ist, zur Speicherung des repräsentativen Werts durch das erste drahtlose Telekommunikationsmittel zu übertragen;
und dadurch, dass er eingerichtet ist, einen Container gemäß in Abhängigkeit mindestens eines Vergleichs zwischen der Anzahl von im Speicher der ersten drahtlosen Telekommunikationsmittel (10) des Containers gespeicherten repräsentativen Werten eines Ergebnisses einer Messung des Parameters und der Anzahl der vorgegebenen Messzeitpunkte zu qualifizieren oder zu dequalifizieren.

12. Behälter nach Anspruch 11, wobei die zweiten drahtlosen Telekommunikationsmittel des Behälters einen RFID-Leser aufweisen, wobei die ersten drahtlosen Telekommunikationsmittel des Containers ein RFID-Etikett aufweisen.

13. Behälter nach Anspruch 11 bis 12, der dazu eingerichtet ist, eine Angabe des vorgegebenen Messzeitpunkts in die Nachricht einzufügen.

14. Behälter nach einem der Ansprüche 11 bis 13, in dem die Messzeitpunkte mit einer Periode der Länge T beabstandet sind.

15. Behälter nach Anspruch 14, wobei T zwischen 10 Sekunden und 6 Stunden liegt.

## Claims

1. A qualification method for a container (5) provided with first wireless telecommunications means (10) comprising a memory (13), said method being **characterized in that** it comprises the following steps when the container is stored in a container storage enclosure (1), said enclosure comprising at least one atmospheric sensor (20) suitable for measuring, within the enclosure, at least one atmospheric parameter from among the temperature, pressure and humidity, and at least second wireless telecommunications means (6):
a/ radiofrequency transmission by the second wireless telecommunications means of the enclosure, to first wireless telecommunications means (10) of the container, of a message including a value representative of a result of a measurement of said parameter in the enclosure; step a/ being reiterated after each measurement moment of a plurality of determined successive measurement moments;
b/ for each of said messages received by the first wireless telecommunications means of the container, storage, by said first wireless telecommunications means of the container, of the representative value included in the received message, the memory of the first wireless telecommunications means of the container including values representative of the results of measurements of said parameter successively stored and each associated with a distinct determined measurement moment;
according to which the method further comprises a step for qualifying or disqualifying the container (5) as a function of at least one comparison between the number of values representative of a result of a measurement of said parameter that are stored in the memory of the first wireless telecommunications means (10) of the container and the number of said determined measurement moments.

2. The method according to claim 1, wherein the first wireless telecommunications means of the container include a RFID tag and the second wireless telecommunications means of the enclosure include a RFID reader.

3. The method according to claim 1 or 2, wherein in step a/, the message further comprises an indication of the moment of said determined measurement and wherein in step b/, said indication is stored matching said representative value.

4. The method according to any one of the preceding claims, wherein steps a/ and b/ are reiterated for each period of length T.

5. The method according to claim 4, wherein T is comprised between 10 seconds and 6 hours.

6. The method according to any one of the preceding claims, wherein the container (5) is qualified or disqualified as a function of a number of determined measurement moments for which the memory (13) of the first wireless telecommunications means (10) of the container does not include a value representative of a measurement result, said measurement moments being situated between the two measurement moments for which representative values of a result have been stored in the memory of the first telecommunications means.

7. The method according to one of the preceding claims, wherein a container (5) is further qualified or disqualified based on the representative values stored in the memory of the first wireless telecommunications means (10) of the container.

8. The method according to one of the preceding claims, comprising the following steps when the container (5) is stored in a container transport enclosure (30), then transported between a departure point and a destination point in said transport enclosure, said transport enclosure comprising a sensor (32) suitable for measuring the value of at least one atmospheric parameter from among the temperature, pressure and humidity within the enclosure and three wireless telecommunications means (31) comprising a memory:
- recording, during transport, a value representative of a result of a measurement of said parameters for each of several successive determined measurement moments, in the memory of the third wireless telecommunications means (31) of the transport enclosure (30);
- transmission, by the third wireless telecommunications means of the transport enclosure (30), said representative values to a processing device comprising fourth wireless telecommunications means and situated at the destination point;
- qualification or disqualification of the container based on said representative values received by the processing device;

9. The method according to claim 8, wherein third wireless telecommunications (31) means comprise a RFID tag and the fourth wireless telecommunications means comprise a RFID reader.

10. The method according to claim 8 or 9, further comprising the following steps:
- transmission, from the fourth wireless telecommunications means of the processing device to the first wireless communication means of the container (5), of at least said representative values received by the processing device;
- reception of said representative values by the first wireless communication means of the container and storage by said first communication means of said parameter values received in the memory.

11. A storage enclosure (1) for containers comprising at least one atmospheric condition sensor (20) suitable for measuring, within the enclosure, at least one atmospheric parameter from among the temperature, pressure and humidity, and said enclosure comprising second wireless telecommunications means (6) suitable for communicating with first wireless telecommunications means of at least one container (5) stored in the enclosure, said storage enclosure being **characterized in that** it is suitable for transmitting, after each measurement moment of a plurality of determined successive measurement moments, to first wireless telecommunications means of said container, a message including a value representative of a result of a measurement of said parameter in the enclosure for the storage, by the first wireless telecommunications means, of the representative value;
and **in that** it is suitable for qualifying or disqualifying a container as a function of at least one comparison between the number of representative values of a result of the measurement of said parameter that are stored in the memory of the first wireless telecommunications means (10) of the container and the number of said determined measurement moments.

12. The enclosure according to claim 11, wherein the second wireless telecommunications means of the enclosure include a RFID reader, and the first wireless telecommunications means of the container include a RFID tag.

13. The enclosure according to claim 11 to 12, suitable for inserting an indication of the time of said determined measurement into the message.

14. The enclosure according to any one of claims 11 to 13, wherein the measurement moments are separated by a period of length T.

15. The enclosure according to claim 14, wherein T is comprised between 10 seconds and 6 hours.
